(19) Europäisches Patentamt
European Patent Office
Office européen des brevets

(11) **EP 1 821 076 A1**

(12) **EUROPEAN PATENT APPLICATION**
published in accordance with Art. 158(3) EPC

(43) Date of publication:
**22.08.2007 Bulletin 2007/34**

(51) Int Cl.:
*G01F 1/28* (2006.01)    *G01F 1/00* (2006.01)

(21) Application number: **05811470.3**

(22) Date of filing: **29.11.2005**

(86) International application number:
**PCT/JP2005/021865**

(87) International publication number:
**WO 2006/062007 (15.06.2006 Gazette 2006/24)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **09.12.2004 JP 2004356890**

(71) Applicant: **Konica Minolta Medical & Graphic, Inc.
Tokyo 163-0512 (JP)**

(72) Inventor: **WADA, Yasunori
Konica Minolta Medical&Graphic,Inc.
Tokyo 1928505 (JP)**

(74) Representative: **Gille Hrabal Struck Neidlein Prop
Roos
Patentanwälte
Brucknerstrasse 20
40593 Düsseldorf (DE)**

(54) **FLOW RATE MEASUREMENT DEVICE**

(57)    A movable member 13, whose physical change occurs corresponding to respiration flow rate, is disposed in the pipe 11. The pipe 11 and a holder section are structured to be capable of being detachable. When staring respiration with the mouth touching to the pipe 11, the movable member 13 bends corresponding to respiration flow rate. Image data of the movable member 13 is obtained through the pipe 11 by a CCD area sensor disposed outside the pipe 11 and the bending amount is detected. The respiration rate is calculated based on the flow rate data corresponding to the bending amount of the movable member 13 in use.

## FIG. 2 (a)

FIG. 2 (b)

**Description**

**TECHNICAL FIELD**

The present invention relates to a flow rate measurement apparatus for measuring the flow rate in a flowing path, through which fluid flows.

**BACKGROUND OF THE INVENTION**

In the inspection of a patient having respiratory diseases, a lung function inspection is an important inspection as well as image diagnosis including CT (Computed Tomography) and X-ray photography, and blood inspection. The lung function inspection is conducted almost always when conducting diagnosis of the patient having chronic respiratory diseases, such as, pulmonary emphysema, bronchial asthma and bronchiectasis. In recent years, COPD (Chronic Obstructive Pulmonary Disease) has attracted a great deal of attention. The number of latent patients having COPD is estimated to be several millions. The lung function inspection is very important in the inspections for this disease. In the medical checks conducted at physical training gymnastics, measurements of, such as, a lung capacity, tidal volume, forced expiratory volume in 1 second and a residual volume are conducted. The equipment used for these measurements is the respiration flow rate measurement apparatus.

With respect to the respiration flow rate measurement apparatus, there are a method for measuring the density of carbon dioxide by using a Non-Dispersive Infrared Analyzing method (NDIR) and a method for using a heat ray. A pneumotacho sensor for measuring the flow rate based on the pressure difference between the upstream side and the downstream side of the laminar flow resistive element in the path where gas flows is also used (Patent document 1 for example). A respiration flow rate / flow speed measuring apparatus has disclosed for measuring the respiration flow rate and the flow speed based on the rotation speed and the rotation direction caused by the whirlpool flow of a spinning body, which is provided in a flow path between a pair of leaning members generating the whirlpool flow through the exhalation and inhalation (Patent document 2 for example).

However, since the configuration of the apparatus was complicated, the flow rate measurement apparatus of the prior art described above has been often expensive.

In general, there are many cases that the exhalation from an organism includes germs, such as viruses. When a person having a disease to be infected through air uses the respiration flow rate measurement apparatus, there has been a possibility that bacteria, which become an infection source, exist in the path, through which the exhalation passes. Thus, it is recommended that with respect to the measurement apparatus which has been once used, the portion where the exhalation has passed should be sterilized or replaced with a new parts member. However, to sterilize the apparatus every time the apparatus is used is not only troublesome but also expensive because of the expenditure required for the consumptions and disposal of an antiseptic. On the other hand, in order to establish the disposal system where the used material is disposed and the measurement is conducted by using a new material every time when conducting the measurement, it has been necessary to configure the measurement apparatus in a low cost. When a sensor and an expensive material for measuring the respiration flow rate are used in the exhalation path, there has been a problem that to dispose these materials makes the cost high.

As described in Patent document 1, even though it is possible to structure the flow rate measurement apparatus for detecting the pressure difference between the upstream side and the downstream side of the laminar flow resistive element in the path less expensively, it has been necessary to provide a pressure sensor in a fluid flow path. Accordingly, even though when making the main path of the fluid disposal, it is necessary to reuse the portion where the pressure sensor is set without replacing the portion. Thus, there has been a problem when measuring the flow rate of the fluid having toxic or infected fluid.

[Patent document 1] Japanese Patent Publication Open to Public Inspection No. H7-83713
[Patent document 2] Japanese Patent Publication Open to Public Inspection No. 2000-298043

**DISCLOSURE OF THE INVENTION**

An object of the present invention is to simplify the apparatus structure, provide a less expensive flow rate measurement apparatus and secure the safety in view of the problems associated with the prior art described above.

In accordance with one aspect of the present invention, a flow rate measurement apparatus comprises
a pipe, through which fluid flows,
a movable member, whose physical change occurs corresponding to a flow rate of the fluid, the movable member being disposed inside the pipe in a direction so as to block a part of or all of the flow of the fluid,
a detection device for detecting the physical change amount of the movable member without being in contact with the movable member, the detection device being disposed outside the pipe, and

a calculation device for calculating the flow rate of the fluid based on the physical change amount of the movable member detected by the detection device.

**BRIEF DESCRIPTION OF THE DRAWINGS**

Fig. 1 illustrates a schematic configuration of a respiration flow rate measurement apparatus 100 in the first embodiment of the present invention.

Fig. 2(a) illustrates a longitudinal cross sectional view of a pipe 11, which has been cut in an axial direction, Fig. 2(b) illustrates a vertical cross sectional view of a pipe 11, which has been cut in a radial direction.

Fig. 3 illustrates a lateral cross sectional view of the pipe 11 and a holder section 12, which have been cut in an axial direction.

Fig. 4 illustrates a block diagram of the configuration of a PC 20.

Fig. 5 illustrates a flowchart showing a respiration flow rate measuring process executed by the respiration flow rate measurement apparatus 100.

Fig. 6(a) illustrates a longitudinal cross sectional view of a pipe 11a when cutting the pipe 11a of the second embodiment of the present invention in an axial direction, Fig. 6(b) illustrates a vertical cross sectional view when cutting the pipe 11a in a radial direction.

Fig. 7 illustrates another method for attaching a filter 15a onto the pipes 11d and 11e.

Fig. 8(a) is a longitudinal sectional view of the pipe 11b when cutting the pipe 11b of the respiration flow rate measurement apparatus of the third embodiment of the present invention in the axial direction. Fig. 8(b) illustrates the cross sectional view of the pipe 11b when conducting the respiration.

Fig. 9 illustrates a longitudinal cross sectional view of a pipe 11c of the fourth embodiment of the respiration flow rate measurement apparatus of the present invention when cutting the pipe 11c in the axial direction.

Fig. 10 illustrates a graph showing the measurement results of a respiration flow rate measured by the respiration flow rate measurement apparatus 100.

Fig. 11 illustrates a graph showing the measurement results of a respiration flow rate measured by the respiration flow rate measurement apparatus 100.

**DESCRIPTION OF SYMBOLS**

| | |
|---|---|
| 100 | Respiration flow rate measurement apparatus |
| 10 | Measurement section |
| 11, 11a, 11b, 11c | Pipe |
| 12 | Holder section |
| 13, 13a, 13b, and 13c | Movable member |
| 14 | CCD area sensor |
| 15 | Filter |
| 16 | Axis |
| 17 | Spring |
| 18 | Spring |
| 20 | PC |
| 21 | CPU |
| 22 | Operation section |
| 23 | Display section |
| 24 | Data input section |
| 25 | Bar code input section |
| 26 | ROM |
| 27 | RAM |
| 28 | Storing section |
| 30 | Bar code reading device |
| 40 | Bar code |

**DESCRIPTION OF THE PREFERRED EMBODIMENTS**

An object of the present invention can be attained by following configurations.

(1) A flow rate measurement apparatus configured by
a pipe, through which fluid flows,
a movable member, whose physical change occurs corresponding to a flow rate of the fluid, the movable member being disposed inside the pipe in a direction so as to block a part of or all of the flow of the fluid,
a detection device for detecting a physical change amount of the movable member without being in contact with the movable member, the detection device being disposed outside the pipe, and
a calculation device for calculating the flow rate of the fluid based on a physical change amount of the movable member detected by the detection device.
(2) The flow rate measurement apparatus of item 1,
wherein the pipe including the movable member, and the detection device are structured to be detachable.
(3) The flow rate measurement apparatus of item 1 or item 2
wherein the detection device detects the physical change amount of the movable member by detecting an electromagnetic wave having passed through or reflected on the movable member.
(4) The flow rate measurement apparatus of item 3,
wherein the detection device is configured by a CCD area sensor, a CCD line sensor, a CMOS area sensor or a CMOS line sensor.
(5) The flow rate measurement apparatus of item 1 or item 2,
wherein the detection device emits a sound wave and detects the physical change amount of the movable member by detecting the sound wave having passed through or reflected on the movable member.
(6) The flow rate measurement apparatus of item 5,
wherein the sound wave is an ultrasonic wave.
(7) The flow rate measurement apparatus of any one of items 1 - 6,
wherein the movable member is structured of an elastic body or connected with an elastic body.
(8) The flow rate measurement apparatus of any one of items 1 - 7,
wherein one end of the movable member is fixed on an internal wall of the pipe or a portion which is connected to the internal wall.
(9) The flow rate measurement apparatus of any one of items 1 - 8,
wherein the movable member and the pipe including the movable member are structured of the same kind of resin material.
(10) The flow rate measurement apparatus of any one of items 1 - 9,
wherein internal wall of the pipe is structured along a moving locus of an edge section of the movable member, the moving locus being generated by the physical change of the movable member.
(11) The flow rate measurement apparatus of any one of items 1 - 10, further including
a plurality of movable members, and
a storing device for storing flow rate data corresponding to a physical change amount of the respective movable members, the flow rate data being measured in advance,
wherein the calculation device calculates the flow rate of the fluid based on the flow rate data corresponding to a physical change amount of the movable member in use.
(12) The flow rate measurement apparatus of item 11,
wherein the pipe includes identification information for individually identifying each of the plurality of the movable members and the flow rate measurement apparatus has a data specifying device for specifying the flow rate data corresponding to the physical change amount of the movable member based on the identification information.
(13) The flow rate measurement apparatus of any one of items 1 - 12,
wherein the calculation device calculates flow rates of bidirectional flow of the fluid in an axial direction of the pipe.
(14) The flow rate measurement apparatus of any one of items 1 - 13,
wherein the fluid is expiration gas and/or inspiration gas.
(15) The flow rate measurement apparatus of any one of items 1 - 14,
further including,
filters provided on the upstream side and downstream side of the movable member inside the pipe.

Next, details of the methods to solve the above problems will be described as follows.
In order to solve the problems described above, the apparatus of item 1 is characterized by being provided with
a pipe, through which fluid flows,
a movable member, whose physical change occurs corresponding to a flow rate of the fluid, the movable member being disposed inside the pipe in a direction for blocking a part of or all of the flow of the fluid,
a detection device for detecting a physical change amount of the movable member without being in contact with the movable member, the detection device being disposed outside the pipe, and
a calculation device for calculating the flow rate of the fluid based on the physical change amount of the movable member detected by the detection device.

The apparatus described in item 2 is a flow rate measurement apparatus of item 1 characterized in that the pipe including the movable member, and the detection device are structured to be detachable.

The apparatus described in item 3 is a flow rate measurement apparatus of item 1 or item 2 characterized in that the detection device detects the physical change amount of the movable member by detecting an electromagnetic wave having passed through or reflected by the movable member.

The apparatus described in item 4 is a flow rate measurement apparatus of item 3 characterized in that the detection device is configured by a CCD area sensor, a CCD line sensor, a CMOS area sensor or a CMOS line sensor.

The apparatus described in item 5 is a flow rate measurement apparatus of item 1 or a flow rate measurement apparatus of item 2 characterized in that the detection device emits a sound wave and detects the physical change amount of the movable member by detecting the sound wave having passed through or reflected on the movable member.

The apparatus described in item 6 is a flow rate measurement apparatus of item 5 characterized in that the sound wave is an ultrasonic wave.

The apparatus described in item 7 is a flow rate measurement apparatus of any one of items 1 - 6 characterized in that the movable member is structured of an elastic body or connected with an elastic body.

The apparatus described in item 8 is a flow rate measurement apparatus of any one of items 1 - 7 characterized in that one end of the movable member is fixed on an internal wall of the pipe or a portion which is connected to the internal wall.

The apparatus described in item 9 is a flow rate measurement apparatus of any one of items 1 - 8 characterized in that the movable member and the pipe including the movable member are structured of the same kind of resin material.

The apparatus described in item 10 is a flow rate measurement apparatus of any one of items 1 - 9 characterized in that internal wall of the pipe is structured along a moving locus of an edge section of the movable member, the moving locus being generated by the physical change of the movable member.

The apparatus described in item 11 is a flow rate measurement apparatus of any one of items 1 - 10 characterized by including
a plurality of movable members, and
a storing device for storing flow rate data corresponding to the physical change amount of the respective movable members, the flow rate data being measured in advance,
wherein the calculation device calculates the flow rate of the fluid based on the flow rate data corresponding to the physical change amount of the movable member in use.

The apparatus described in item 12 is a flow rate measurement apparatus of item 11 characterized in that the pipe includes identification information for individually identifying each of the plurality of the movable members and the apparatus has a data specifying device for specifying the flow rate data corresponding to the physical change amount of the movable member based on the identification information.

The apparatus described in item 13 is a flow rate measurement apparatus of any one of items 1 - 12 characterized in that the calculation device calculates flow rates of bidirectional flow of the fluid in an axial direction of the pipe.

The apparatus described in item 14 is a flow rate measurement apparatus of any one of items 1 - 13 characterized in that the fluid is expiration gas and/or inspiration gas.

The apparatus described in item 15 is a flow rate measurement apparatus of any one of items 1 - 14 characterized by further including filters provided on the upstream side and downstream side of the movable member inside the pipe.

Next, the effects of the present invention will be described.

According to the apparatus described the item 1, since the detection device disposed outside the pipe detects the physical change amount of the movable member disposed so as to block a part of or all of the flow of the fluid in the pipe, without being contact with the movable member, the physical change being generated corresponding to the flow rate of the fluid, and calculates the flow rate of the fluid, the apparatus structure becomes simple and a less expensive flow rate measurement apparatus can be provided. Further, since the detection device is disposed outside the pipe, it becomes easy to sterilize and change the pipe every time the flow rate measurement apparatus is used. Accordingly, when dealing with the fluid including toxic or infected fluid, the safety can be secured.

According to the apparatus described in the item 2, since the movable member and the detection device are configured so as to be detachable, the pipe can be easily replaced and the safety can be secured when dealing with the fluid including toxic and infected fluid.

According to the apparatus described in the item 3, the physical change amount can be detected by detecting the electromagnetic waves, which have passed through or reflected on the movable member.

According to the apparatus described in the item 4, the physical change amount of the movable member can be detected by using the CCD area sensor, the CCD line sensor, CMOS area sensor or the CMOS line sensor.

According to the apparatus described in the item 5, the physical change amount of the movable member can be detected by emitting sound waves to the movable member and detecting the sound waves having passed through or reflected on the movable member.

According to the apparatus described in the item 6, the physical change amount of the movable member can be detected by detecting the ultrasonic wave.

According to the apparatus described in the item 7, since the movable member is structured of the elastic body or connected with the elastic body, the physical changes can be generated corresponding to the flow rate of the fluid.

According to the apparatus described in the item 8, since the one end of the movable member is fixed on the internal wall or a portion connected to the pipe, the physical changes can be generated while setting the one end as a fulcrum.

According to the apparatus described in the item 9, since the movable member and the pipe including the movable member are structured of the same kind of resin material, the treatment after use becomes easy.

According to the apparatus described in the item 10, since the inside wall of the pipe is structured so as to be along the moving locus of the edge section of the movable member, the moving locus being generated by the physical changes of the movable member, it can be prevented that the physical change amount of the movable member compared with a flow rate change becomes small when the flow rate becomes large.

According to the apparatus described in the item 11, since the flow rate data corresponding to the physical change amount of the respective movable members of a plurality of the movable members, the flow rate data having been measured in advance, is stored in the storing device, and the flow rate of the fluid is calculated based on the flow rate data corresponding to the physical change amount of the movable member in use, the flow rate can be calculated corresponding to each movable member.

According to the apparatus described in the item 12, since the identification information for individually identifying each of the plurality of the movable members is included in the pipe and the flow rate data corresponding to the physical change amount of the movable member is specified based on the identification information, it becomes possible to prevent data input errors and the flow rates can be calculated corresponding to respective movable members.

According to the apparatus described in the item 13, since the flow rates of bidirectional flow of the fluid in the axial direction of the pipe are calculated, the apparatus structure becomes simple and a less expensive flow rate measurement apparatus can be provided.

According to the apparatus described in the item 14, it becomes possible to simplify the apparatus configuration of the flow rate measurement apparatus for measuring the flow rate of expiration gas and/or inspiration gas, to provide a less expensive flow rate measuring apparatus and to secure the safety.

According to the apparatus described in the item 15, floating objects, such as dust is intercepted and at the same time the local flow of the fluid can be removed by providing the filter in the upstream side and the downstream side of the movable member inside the pipe.

[FIRST EMBODIMENT]

The first embodiment of the present invention will be described by referring to drawings. However, the present invention is not limited to the examples illustrated in Figures.

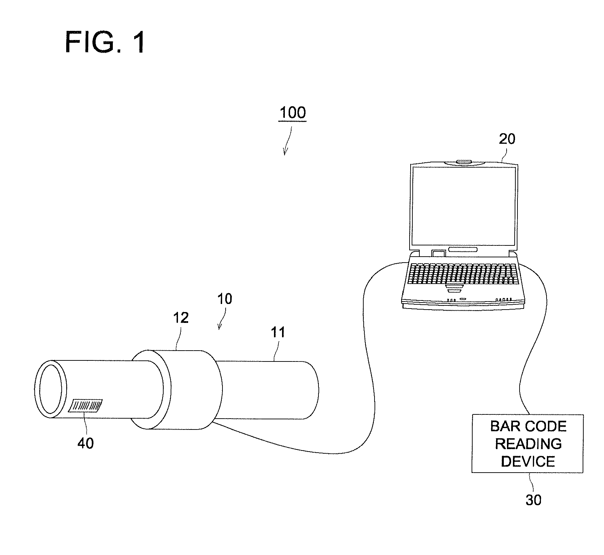

Fig. 1 illustrates a schematic configuration of the respiration flow rate measurement apparatus 100 of the first embodiment. The respiration flow rate measurement apparatus 100 is configured by a measurement section 10, a PC (Personal Computer) 20 and a bar code reading device 30.

The measurement section 10 includes a pipe 11 and a holder section 12. The pipe 11 is configured by a cylindrically structured transparent resin and the pipe 11 forms a path, through which respiration gas flows. The pipe 11 and the holder section 12 are structured to be detachable. The holder section 12 and the PC 20 are directly connected or connected through a network.

Further, a bar code 40 is adhered onto the pipe 11 as identification information for individually identifying a movable member 13, which will be described later.

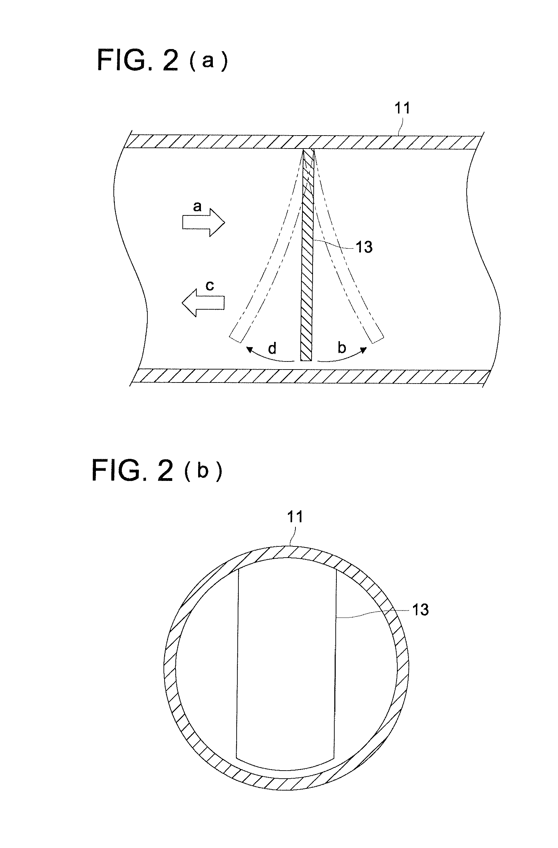

Fig. 2(a) illustrates a longitudinal cross sectional view of a pipe 11, which has been cut in the axial direction, and Fig. 2(b) illustrates a vertical cross sectional view of the pipe 11, which has been cut in the radial direction. As shown in Figs. 2(a) and 2(b), in the pipe, a movable member 13 is disposed so as to block a part of the respiration gas flow. One end of the movable member 13 is fixed onto the inside wall of the pipe 11. The movable member 13 is structured of an elastic body, such as elastic resin having a bending characteristic and bends corresponding to the flow rate of respiration gas. The degree of the bend is decided by the elastic force, the open degree of a flow path caused by transformation, and the change of gas flow. The elastic force is decided by the thickness and the shape of the elastic body 13 and the quality of the material. However, it is possible to set the bending amount constant corresponding to a flow rate when the predetermined flow rate of gas flow is set. Further, it is preferable that the movable member 13 and the pipe 11 are made of the same kind of resin material. Here, the same kind denotes that a resin material, to which the same mark can be applied as a recycle mark.

For example, when measuring the exhalation flow rate, in case blowing the exhalation into the pipe 11, gas flow occurs in the arrow direction "a" as shown in Fig. 2(a). Based on this flow, the movable member 13 bends toward the arrow direction "b". When measuring the inhalation flow rate, the gas flow in the arrow direction "c" of Fig. 2(a) occurs by inhaling the inhalation from the pipe 11. Based on this flow, the movable member 13 bends toward the arrow direction "d". The measurement of the respiration flow rate becomes possible by detecting the physical change amount of the movable member 13, namely, the bending amount of the movable member 13. In this invention, the physical changes denote not only the transformation of the shape of a material but also the displacement caused by the shift or the rotation of material.

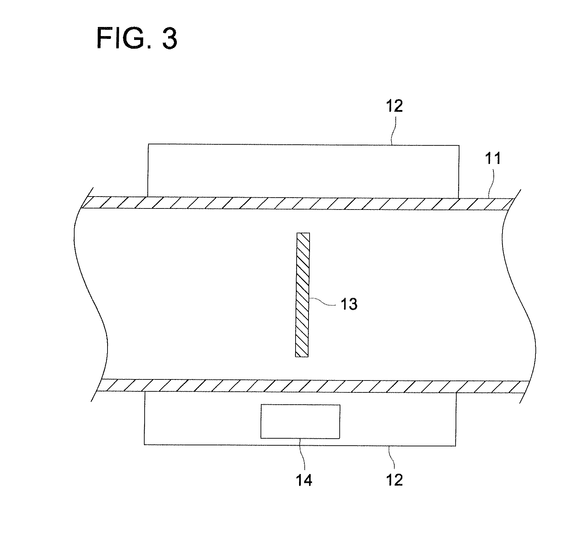

Fig. 3 illustrates a lateral cross sectional view of the pipe 11 and a holder section 12, which have been cut in an axial direction. As shown in Fig. 3, a CCD area sensor 14 for capturing the image of the movable member 13 through a transparent pipe 11 is provided in the holder section 12. The CCD area sensor 14 optically detects the bending amount of the movable member 13.

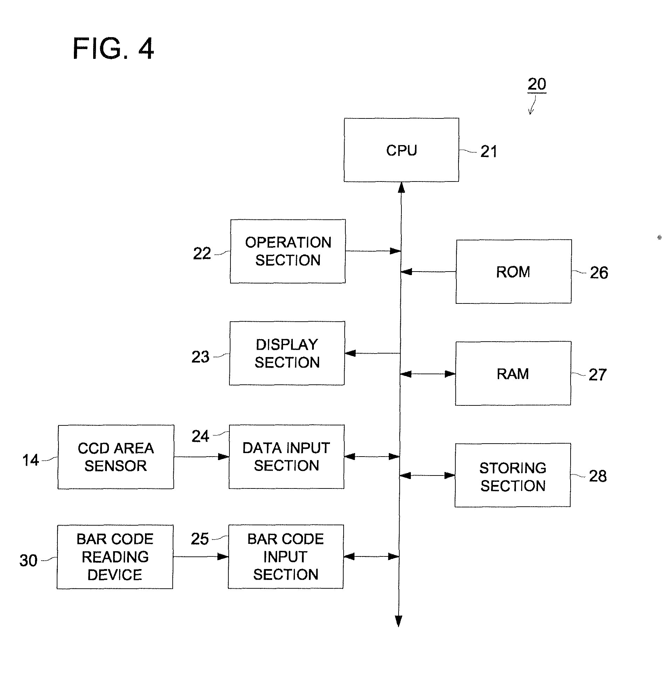

Fig. 4 illustrates a block diagram of the configuration of a PC 20. As shown in Fig. 4, The PC 20 comprises a CPU (Central Processing Unit) 21, an operation section 22, a display section 23, a data input section 24, a bar code input section 25, a ROM (Read Only Memory) 26, a RAM (Random Access Memory) 27 and a storing section 28. The PC 20 may be a PDA (Personal Digital Assistance).

The CPU 21 expands the specified program out of various programs stored in the ROM 26 into the work area of the RAM 27 according to various instructions inputted from operation section 22, executes various processes in cooperation with the program described above and stores the processing results into the predetermined area of the RAM 27.

Concretely, the CPU 21 specifies the flow rate data corresponding to the bending amount of the movable member 13 stored in the memory 28 based on the bar code information read by the bar code reading device 30.

The CPU 21 calculates the flow rate of the respiration gas based on the bending amount of movable member 13 obtained by analyzing the image of the image data outputted from the CCD area sensor 14. At this moment, the CPU 21 calculates the flow rate of the respiration gas based on the flow rate data corresponding to the bend amount of movable member 13 specified based on the bar code information. Since the displacement directions of the movable member 13 in the exhalation and inhalation are opposite each other, it is possible to calculate the flow rates of the bidirectional flows in an axial direction of the pipe 11.

The operation section 22 includes a keyboard having numeric and alphabetic character input keys and various keys and a pointing device, such as a mouse. The operation section 22 outputs the pushdown signals generated by pushing down the keys of the keyboard and operation signals generated by the mouse to the CPU 21.

The display section 23 is configured by a LCD (Liquid Crystal Display) or a CRT (Cathode Ray Tube). The display section 23 displays the operation sequence and the processing results.

The data input section 24 outputs the image data outputted from the CCD area sensor 14 to the CPU 21.

The bar code input section 25 outputs the bar code information read from the bar code adhered on the pipe 11 by the bar code reading device 30 to the CPU 21.

The ROM 26 is configured by non-volatile semiconductor memory. The ROM 26 stores various programs executed by the CPU 21 and data.

The RAM 27 is configured by a re-writable semiconductor element. The RAM 27 is a memory media for temporally storing data. The RAM 27 is configured by a program area for expanding programs to be executed by the CPU 21 and a data area for storing the data inputted from the operation section 22 and the various kinds of processing results of the CPU 21.

The storing section 28 is configured by a HDD (Hard Disk Drive), which stores the flow rate data corresponding to the bending amount of respective movable members 13 of the plurality of movable members 13, which have been measured in advance, and bar code information corresponding to the respective movable members 13. With respect to the flow rate data corresponding to the bending amount of each movable member 13, the flow rate data, which has been measured in advance, is directly inputted from the operation section 22 or from a measurement apparatus and stored in the storing section 28.

The bar code reading device 30 reads the bar code information from the bar code 40 (refer to Fig. 1) adhered on the pipe 11.

Next, the operation of the respiration flow rate measurement apparatus 100 of the first embodiment will be described.

As a basis for the description of the operation, it is assumed that the program for realizing the process described in the flowchart is stored in the ROM 26 as program codes, which can be read by the CPU 21 and the CPU 21 sequentially executes the operations according to the program codes.

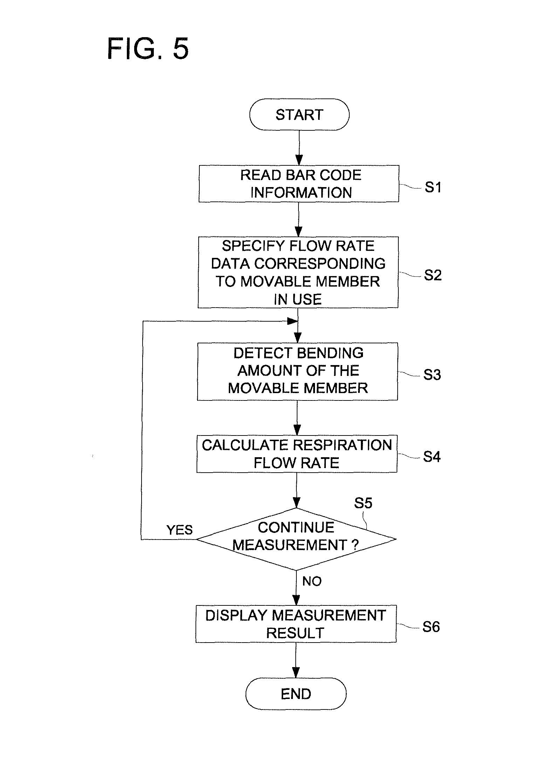

Fig. 5 illustrates a flowchart showing a respiration flow rate measuring process executed by the respiration flow rate measurement apparatus 100.

Firstly, the bar code reading device 30 reads the bar code information from the bar code 40 adhered on the pipe 11 (Step S1). The bar code information is stored in the storing section 28.

Next, the flow rate data corresponding to the bending amount of the movable member 13 in use is specified from the flow rate data stored in the storing section 28 based on the read bar code information (Step S2).

When a user holds the measurement section 10 in his or her hand and starts respiration with the pipe 11 contacted with the mouth, the movable member 13 bends corresponding to the respiration flow rate. The CCD area sensor 14 obtains the image data of the movable member 13 through the pipe 11 and detects the bending amount (Step S3). Then, the respiration flow rate is calculated based on the flow rate data corresponding to the bending amount of the movable member 13 in use (Step S4).

Here, when the measurement of the respiration flow rate is continued (Step S5: YES), the process returns to the Step S3. When the measurement of the respiration flow rate is not continued (Step S5: NO), the measurement results of the respiration flow rate are displayed on the display section 23 (Step S6).

Then, the respiration flow rate measurement process ends.

According to the first embodiment, since the CCD area sensor 14 disposed outside the pipe 11 detects the bending amount of the movable member 13 corresponding to the respiration flow rate without being in contact with the movable member 13, the movable member 13 being disposed in the pipe 11 so as to block a part of respiration gas flow and the respiration flow rate can be calculated based on the detected bending amount of the movable member 13, the structure of the apparatus becomes simple and a less expensive flow rate measurement apparatus can be provided. Further, since the CCD area sensor 14 is disposed outside the pipe 11, it becomes easy to sterilize or replace the pipe every time the respiration flow rate measurement apparatus 100 is used. When handling the fluid including toxicity and infectiveness, safety can be secured.

Further, since the pipe 11 including the movable member 13 and the holder section 12 are configured so as to be detachable, the pipe 11 can be easily changed. Even when there is a patient having an infectious disease, the infection between users can be prevented and safety can be secured.

Further, the treatment after use becomes easy by configuring the movable member 13 and the pipe 11 including the movable member 13 of the same kind of resin material. Since the respiration flow rate measurement apparatus 100 is used for measuring the respiration flow rate, the pipe 11 including the movable member 13 is preferably replaced every time the measurement is conducted. The treatment when disposing of the pipe 11 after use becomes easy.

Further, since the flow rate data corresponding to the bending amount, which has been measured for each of the plurality of movable members 13 in advance, is stored in the storing section 28 and the respiration flow rate is calculated based on the flow rate data corresponding to the bending amount of respective movable members 13 in use, the flow rates can be calculated according to respective movable members 13. Since by adhering the bar code on each pipe 11 for individually identifying respective movable members 13, the flow rate data corresponding to the bending amount of the movable member 13 can be specified based on the bar code, data input errors can be prevented. Further, various calculation processes may be conducted based on the flow rate data, various parameters may be calculated and the respiration flow rate, which has been obtained by a measurement, may be stored in database.


[SECOND EMBODIMENT]

Next, the second embodiment of the present invention will be described.

The respiration flow rate measurement apparatus of the second embodiment is structured by a pipe 11a and a movable member 13a instead of the pipe 11 and the movable member 13 of the first embodiment. The other structures are the same as the first embodiment. Accordingly, the drawings and the descriptions will be omitted. The characterized structure associated with the second embodiment will be described below.

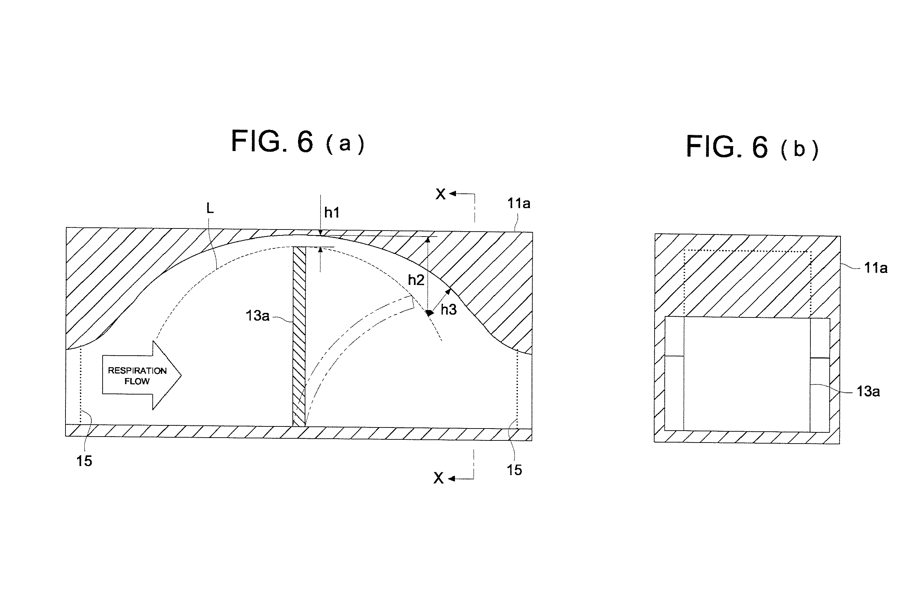

Fig. 6(a) illustrates a longitudinal cross sectional view of a pipe 11a when cutting the pipe 11a in the second embodiment of the present invention in an axial direction. Fig. 6(b) illustrates a vertical cross sectional view when cutting the pipe 11a in a radius direction at X - X line of Fig. 6(a). In the first embodiment, the top portion of the movable member 13 is fixed inside the pipe 11. However, in the second embodiment, the lower portion of the movable member 13a is fixed inside the pipe 11a. As shown in Fig. 6(b), the cross sections of the pipe 11a which are cut in the radius direction and the movable member 13a have a rectangular shape.

Further, as shown in Fig. 6(a), the inside wall of the pipe 11a is structured so as to be along the moving locus of the top edge of the movable member 13a, namely, the inside wall of the pipe 11a is structured so that the change of the cross sectional area of the flow path corresponding to the transformation of the movable member 13a becomes small. When the cross sectional area of the flow path of the pipe 11a forming the flow path, is constant, if the respiration flow rate is small, no problem is caused. However, when the respiration flow becomes large, since the cross sectional area of the flow path rapidly becomes large corresponding to the transformation of the movable member 13a, the bending amount of the movable member 13a corresponding to the flow rate becomes small because the force caused on the movable member 13a becomes small.

For example, as shown in Fig. 6(a), when there is no respiration, the space between the top portion of the movable member 13a and the inside wall of the pipe 11a is h1. In the case where the cross sectional area of the pipe 11a forming the flow path is constant, when the movable member 13a bends, the space between the top portion of the movable member 13a and the inside wall of the pipe 11a becomes h2. By setting the space between the top portion of the movable member 13a and the inside wall of the pipe 11a to h3 in the situation where the movable member 13a bends, in order to suppress the change of the cross sectional area of the flow path corresponding to the transformation of the movable member 13a, it becomes possible to precisely measure the bending amount of the movable member 13a and to correspond a wide range of flow rate.

As shown in Fig. 6(a), filters 15 are provided at the upstream side and the downstream side of the movable member 13a inside the pipe 11a. When gas flow is unbalanced, unevenness occurs in the force against the movable member 13a and it causes the unevenness of the movement of the movable member 13a with respect to the flow rate. Accordingly, in order to remove the local gas flow, the filters 15 for playing a role in regulating the gas flow is preferably provided. Further, in order not to intake floating objects in the air into the body, the filters 15 are useful. With respect to the filters 15, a material having many microscopic holes on the surface can be used. Concretely, a resin film having holes may be used or preferably nonwoven fabric is used. Since the nonwoven fabric does not include dust, the cost is low and the filtering effect is high, the nonwoven fabric is highly preferable.

Regarding the method of detecting the bending amount of the movable member 13a by CCD area sensor 14 and the method of calculation of respiration flow based on the bending amount, the description will be omitted because they are similar to the first embodiment.

According to the second embodiment, since the inside wall of the pipe is structured so as to be along the moving locus L of the end portion of the movable member 13a based on the transformation of the movable member 13a, when the flow rate becomes large, it becomes possible to prevent the bending amount relative to the flow rate from becoming small when the flow rate becomes large.

Further, by providing the filters 15 at the upstream side and the downstream side of the movable member 13a inside the pipe 11a, floating objects in the air, such as dust, can be blocked and local gas flow can be removed.

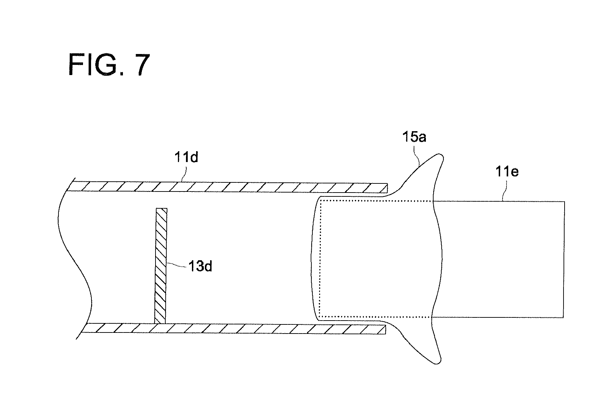

As shown in Fig. 7, the filter 15a may be inserted between the space formed by a pipe 11d and a pipe 11e which is a little smaller in diameter than that of the pipe 11d. Based on this, dust in the respiration gas flowing in the pipe 11d which includes the movable member 13d can be blocked and local gas flow can be removed.

[THIRD EMBODIMENT]

Next, the third embodiment of the present invention will be described.

The respiration flow rate measurement apparatus of the third embodiment is structured by a pipe 11b and a movable member 13b instead of the pipe 11 and the movable member 13 of the first embodiment. The other structures are the same as the first embodiment. Accordingly, the same symbol will be used for the same part of the structure, and the drawings and the descriptions will be omitted. The characterized structure of the third embodiment will be described below.

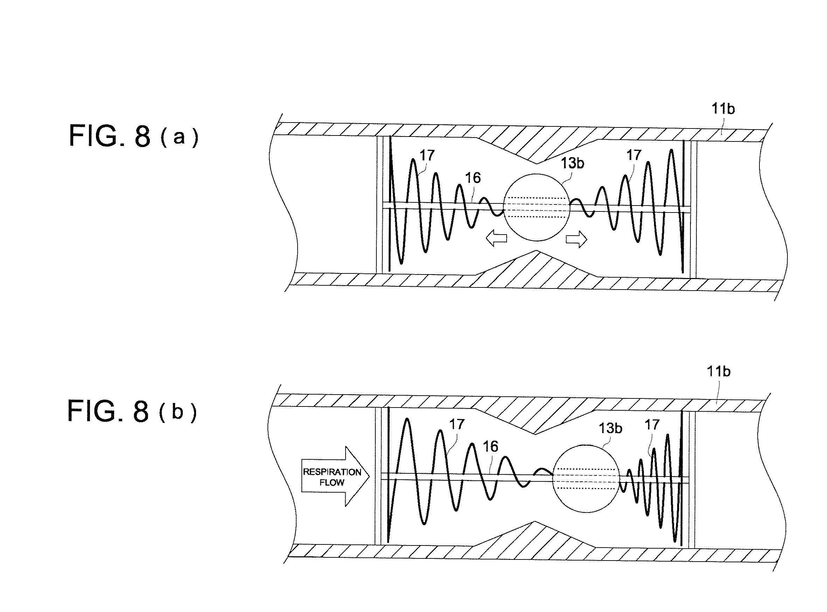

Fig. 8(a) illustrates a longitudinal cross sectional view of a pipe 11b when cutting the pipe 11b of the third embodiment of the present invention in an axial direction. As shown in Fig. 8(a), the movable member 13b having a spherical shape is arranged to move along the shaft 16, which is passed through the hole structured at the center of the spherical movable member 13b. The movable member 13b is connected with springs 17 of elastic bodies, and the position of the movable member 13b is set by the springs 17.

When respiration is conducted, as shown in Fig. 8(b), the movable member 13b moves inside the pipe 11b. Since when the user breathes out air and when the user breathes in air, the directions of the air flow are different, the measurement of the flow rate of the respiration gas of both directions in the axial direction of the pipe 11b can be conducted based on the moving direction of the movable member 13b.

The flow rate data corresponding to the displacement amount of the movable members 13b, which have been measured in advance for the plurality of the respective movable members 13b and the bar code information corresponding to the respective movable members 13b are stored in the storing section 28 of the PC 20.

With respect to the detection method for detecting the displacement amount of the movable member 13b by using the CCD area sensor 14 and the method for calculating the respiration flow rate based on the displacement amount, since the methods are the same as the first embodiment, the description will be omitted.

According to the third embodiment, since the CCD area sensor 14 disposed outside the pipe 11b detects the displacement amount of the movable member 13b disposed inside the pipe 11b, which moves corresponding to the flow rate of the respiration gas, the flow rate is calculated based on the detected displacement amount of the movable member, the structure of the apparatus becomes simple, and a less expensive flow rate measurement apparatus can be provided. Further, since the pipe 11b including the movable member 13b and the holder section 12 are configured so as to be detachable, the pipe 11b can be easily changed. Even when there is a patient having an infectious disease, the infection between users can be prevented and safety can be secured.

[FOURTH EMBODIMENT]

Next, the fourth embodiment of the present invention will be described.

The respiration flow rate measurement apparatus of the fourth embodiment is structured by a pipe 11c and a movable member 13c instead of the pipe 11 and the movable member 13 of the first embodiment. The other structures are the same as the first embodiment. Accordingly, the same symbol will be used for the same part of the structure, and the drawings and the descriptions will be omitted. The characterized structure of the fourth embodiment will be described below.

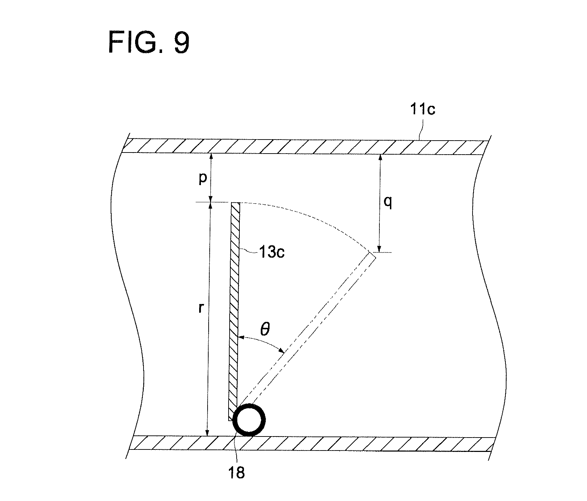

Fig. 9 illustrates a longitudinal cross sectional view of a pipe 11c in the fourth embodiment of the respiration flow rate measurement apparatus of the present invention when cutting the pipe 11c in an axial direction. Here, the cross sections of the pipe 11c in the radius direction and the movable member 13c respectively have rectangular shapes. As shown in Fig. 9, one end of the movable member 13c is connected with a spring 18 and the movable member 13c is disposed inside the pipe 11c. The movable member 13c is structured by a rigid body and arranged to rotate corresponding to the respiration flow rate with the spring 18 being a center. Accordingly, the respiration flow rate can be calculated by detecting the displacement amount of the movable member 13c.

In Fig. 9, when the length of the movable member 13c is "r", the space between the top portion of the movable member 13c and the internal wall of the pipe 11c under the state where the respiration is not conducted is "p" and the space between the top portion of the movable member 13c and the internal wall of the pipe 11c under the state where the movable member 13c leans at an angle of θ is "q", the value of "q" can be obtained by following formula (1).

$$q = p + r (1 - \cos\theta) \qquad\qquad (1)$$

As shown in the formula (1), as the angel 8, which is a leaning angle of the movable member 13c, becomes larger, the space between the top section of the movable member 13c and the internal wall of the pipe 11c becomes larger. As described above, when the flow path is not regulated, as the flow speed increases, the movable member 13c moves in the direction so as to increase the flow path. As a result, the stress caused to the movable member 13c becomes small. Thus, when the flow path becomes larger, even though the flow speed increase, the stress caused to the movable member 13c is small, and the detection accuracy of the displacement of the movable member 13c becomes worse. Accordingly, when it is necessary to measure the large flow rate, the internal wall of the pipe 11c may be designed so as to be along the moving locus of the edge section of the movable member 13c with the moving locus being shaped based on the displacement of the movable member 13c. Namely, the internal wall of the pipe 11c may be designed so as to suppress the expansion of the flow path, when the measurement of the large flow rate is necessary.

In the storing section 28 of the PC 20, the flow rate data corresponding to the displacement amount of the movable member 13c, which has been measured for respective movable members 13c of a plurality of the movable members 13c in advance, and the bar code information corresponding to the respective movable members 13c are stored.

With respect to the detection method for detecting the displacement amount of the movable member 13c by using the CCD area sensor 14, and the method for calculating the respiration flow rate based on the displacement amount, since the methods are the same as the first embodiment, the description will be omitted.

According to the fourth embodiment, since the CCD area sensor 14 disposed outside the pipe 11c detects the displacement amount of the movable member 13c disposed inside the pipe 11c, which moves corresponding to the flow rate of the respiration gas, and the flow rate is calculated based on the detected displacement amount of the movable member, the structure of the apparatus becomes simple, and a less expensive flow rate measurement apparatus can be provided. Further, since the pipe 11c including the movable member 13c and the holder section 12 are configured so as to be detachable, the pipe 11c can be easily changed. Even when there is a patient having an infectious disease, the infection between users can be prevented and safety can be secured.

The description of the respective embodiment described above is an example of a preferable embodiment of the present invention and should not be limited hereto. Various changes relating to detailed structures and detailed movements may be made without departing from the scope of the invention.

In the embodiments described above, the examples, in which the physical change amount of the movable member disposed inside the pipe is detected by the CCD area sensor from outside the pipe, have been described. The movable member may be detected by the CCD area sensor, CCD line sensor, CMOS area sensor, the CMOS line sensor or a photomultiplier with lights having been passed through the movable member, or having been reflected, absorbed or dispersed by the movable member, or lights not having passed through the movable member, or not having been reflected, absorbed or dispersed by the movable member. Since the method for optically detecting the physical change amount of the movable member is relatively low cost for structuring the apparatus, the optical method is preferable. The method for obtaining the image of a part or all of the movable member may be used. The method for detecting the displacement of the movable member by detecting the position of the reflected light when irradiating the laser light or the like and the method for detecting the displacement by measuring the wavelength of the dispersed lights according to the principle of a prism when irradiating white light may be used as a method for detecting the displacement.

It is also possible to detect the physical change amount of the movable member by detecting the electromagnetic wave other than lights by measuring the intensity of electric field or magnetic field. The flow rate measurement apparatus may include the source for generating electromagnetic waves. However, when the source for generating electromagnetic waves is provided outside the flow rate measurement apparatus, it is not necessary to provide the source for generating electromagnetic waves inside the apparatus.

The physical change amount of the movable member may be detected by emitting the sound wave to the movable member and detecting the sound waves having passed through or reflected on the movable member. The sound wave is not limited to an audible range, but it may be an ultrasonic wave.

In the respective embodiments described above, transparent pipe has been used. However, the pipe, through which electromagnetic wave or sound wave which is used to detect physical change amount of the movable member can pass, may be used.

The detection accuracy relative to the time resolution can be improved by generating electromagnetic waves or sound waves for detecting the physical change amount of the movable member in a pulse waveform. For example, when detecting the physical change amount of the movable member by using a CCD or a CMOS, since the movable member moves, the obtained data is unstable and it becomes difficult to detect the physical change amount of the movable member. In this case, it becomes possible to precisely detect the physical change amount of the movable member by using the lights in a pulse waveform having a short time interval.

In the respective embodiments described above, the apparatus for measuring the flow rate of the respiration gas has been described. However, the fluid, which is the object to be measured, may be other kind of fluid. Since the pipe including a movable member is detachable from other part of the apparatus, when handling the fluid having toxicity or an infection character, safety can be secured.

In the respective embodiments described above, an example using a bar code as identification information has been described. However, other kind of identification information may be used.

The material and the elasticity factor of the movable member in this invention may be arbitrarily selected based on the character of the fluid to be measured or the flow rate range to be measured. When measuring the respiration flow rate, since moisture is included in exhalation, the material, which is not swollen by the moisture, is preferable. For example, resin such as PET (Polyethylen terephthalate), Polyethylen, Polypropylene or Polyvinyl chloride, and a metal plate spring may be preferably used.

In the measurement of this invention, the relationship between the cross section area of the movable member and the cross section area of the flow path gives the effects on the measurable range and measurement accuracy. The relationship between them can be arbitrarily adjusted in response to the object. In the case of measurement of respiration flow rate, according to JIS ,Japanese Industrial Standards, (T1170 - 1987), the measurable range is 0.3 L - 12.0 L. The flow path suitable for this measurement is one which has the cross sectional area of 4 cm$^2$ - 100 cm$^2$, preferably 5 cm$^2$ - 25 cm$^2$. With respect to the movable member, when using PET, the thickness is preferably 0.1 mm - 0.5 mm, more preferably 0.2 - 0.4 mm.

<EXPERIMENTAL EXAMPLE>

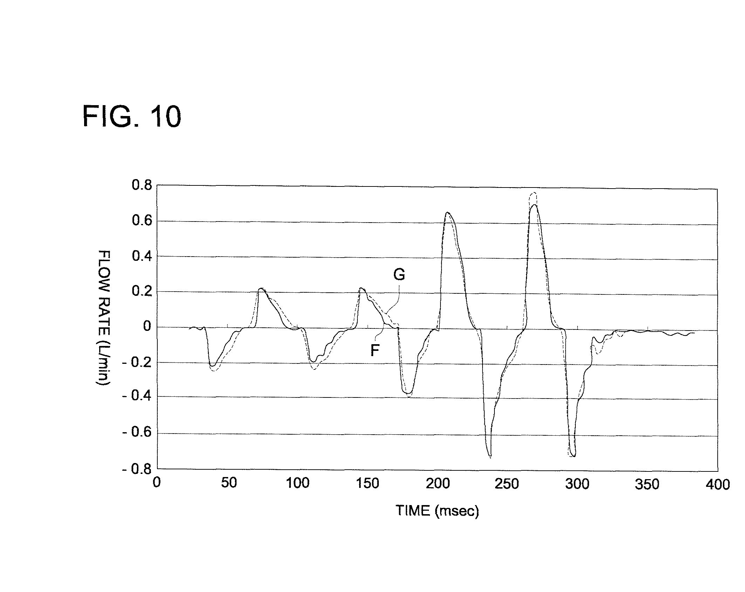

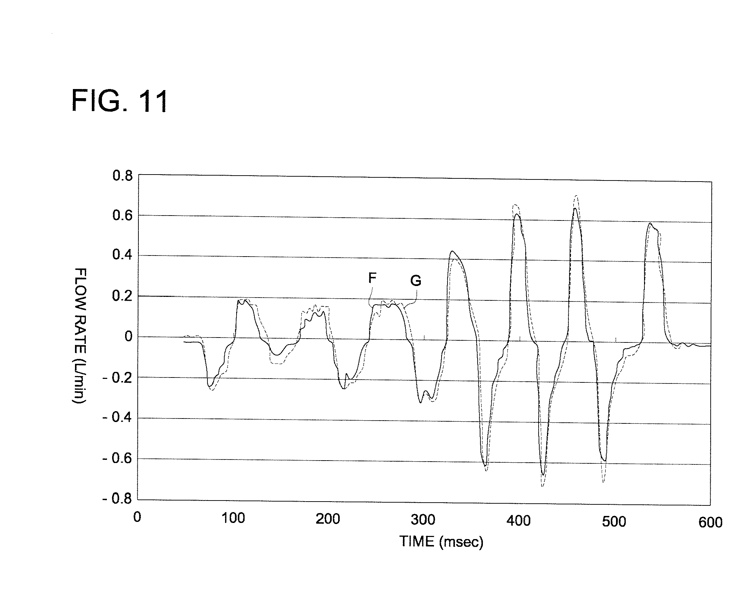

Figs. 10 and 11 illustrate the measurement results of a respiration flow rate measured by the respiration flow rate measurement apparatus 100 described in the first embodiment. The bending amount of the movable member is detected by analyzing the image data obtained by photographing the movable member 13 using the CCD area senor 14. Then the flow rate is calculated based on the detected result. The result F of the calculated result is shown by a solid line. As a reference, the measurement result G of the flow rate measured at the same time by a spirometer, which has been conventionally used, will be shown. In Figs. 10 and 11, the unit of the lateral axis is time (msec) and the unit of the vertical axis is a flow rate (L / min).

When comparing the measurement result F of the respiration flow rate measurement apparatus 100 with the measurement result G of a conventional spirometer, there was a significantly positive correlation, which was 97% correlation.

**Claims**

1. A flow rate measurement apparatus comprising:

   a pipe, through which fluid flows;
   a movable member whose physical change occurs corresponding to a flow rate of the fluid, the movable member being disposed inside the pipe in a direction so as to block a part of or all of a flow of the fluid;
   a detection device for detecting an amount of the physical change of the movable member without being in contact with the movable member, the detection device being disposed outside the pipe; and
   a calculation device for calculating the flow rate of the fluid based on the physical change amount of the movable member detected by the detection device.

2. The flow rate measurement apparatus of claim 1,
   wherein the pipe including the movable member, and the detection device are structured to be detachable.

3. The flow rate measurement apparatus of claim 1,
   wherein the detection device detects the physical change amount of the movable member by detecting an electromagnetic wave having passed through or reflected on the movable member.

4. The flow rate measurement apparatus of claim 3,
   wherein the detection device is configured by a CCD area sensor, a CCD line sensor, a CMOS area sensor or a CMOS line sensor.

5. The flow rate measurement apparatus of claim 1,
   wherein the detection device detects the physical change amount of the movable member by detecting a sound wave having passed through or reflected on the movable member after emitting the sound wave to the movable member.

6. The flow rate measurement apparatus of claim 5,
   wherein the sound wave is an ultrasonic wave.

7. The flow rate measurement apparatus of claim 1,
   wherein the movable member is made of an elastic body or connected with an elastic body.

8. The flow rate measurement apparatus of claim 1,
   wherein one end of the movable member is fixed on an internal wall of the pipe or a portion which is connected to the internal wall.

9. The flow rate measurement apparatus of claim 1,
   wherein the movable member and the pipe including the movable member are made of a same kind of resin material.

10. The flow rate measurement apparatus of claim 1,
    wherein an internal wall of the pipe is structured along a moving locus of an edge section of the movable member, the moving locus being formed by the physical change of the movable member.

11. The flow rate measurement apparatus of claim 1, further comprising:

    a plurality of movable members; and
    a storing device for storing flow rate data corresponding to the physical change amount of each of the plurality of movable members, the flow rate data being measured in advance;

    wherein the calculation device calculates the flow rate of the fluid based on the flow rate data corresponding to the physical change amount of the movable member in use.

12. The flow rate measurement apparatus of claim 11,
    wherein the pipe includes identification information for individually identifying each of the plurality of the movable members and the flow rate measurement apparatus comprising
    a data specifying device for specifying flow rate data corresponding to the physical change amount of the movable member based on the identification information.

13. The flow rate measurement apparatus of claim 1,
    wherein the calculation device calculates flow rates of bidirectional flow of the fluid in an axial direction of the pipe.

14. The flow rate measurement apparatus of claim 1,
    wherein the fluid is at least one of expiration gas and inspiration gas.

15. The flow rate measurement apparatus of claim 1, further comprising:

    filters provided on an upstream side and a downstream side of the movable member inside the pipe.

# FIG. 1

FIG. 2 ( a )

FIG. 2 ( b )

FIG. 3

# FIG. 4

20

| | | |
|---|---|---|
| | **CPU** — 21 | |
| 22 — OPERATION SECTION | | ROM — 26 |
| 23 — DISPLAY SECTION | | RAM — 27 |
| 14 — CCD AREA SENSOR | 24 — DATA INPUT SECTION | STORING SECTION — 28 |
| 30 — BAR CODE READING DEVICE | 25 — BAR CODE INPUT SECTION | |

# FIG. 5

```
              ┌──────────┐
              │  START   │
              └──────────┘
                   │
                   ▼
         ┌────────────────────┐
         │   READ BAR CODE    │───  S1
         │    INFORMATION     │
         └────────────────────┘
                   │
                   ▼
         ┌────────────────────┐
         │ SPECIFY FLOW RATE  │
         │ DATA CORRESPONDING │───  S2
         │ TO MOVABLE MEMBER  │
         │      IN USE        │
         └────────────────────┘
                   │
                   ▼
         ┌────────────────────┐
         │  DETECT BENDING    │
         │   AMOUNT OF THE    │───  S3
         │  MOVABLE MEMBER    │
         └────────────────────┘
                   │
                   ▼
         ┌────────────────────┐
         │ CALCULATE RESPIRATION │───  S4
         │    FLOW RATE       │
         └────────────────────┘
                   │        S5
                   ▼
    YES      ╱──────────────╲
  ◄─────────┤   CONTINUE     │
            │ MEASUREMENT ?  │
             ╲──────────────╱
                   │
                   │ NO
                   ▼
         ┌────────────────────┐
         │ DISPLAY MEASUREMENT │───  S6
         │      RESULT        │
         └────────────────────┘
                   │
                   ▼
              ┌──────────┐
              │   END    │
              └──────────┘
```

FIG. 6 (a)

FIG. 6 (b)

EP 1 821 076 A1

# FIG. 7

FIG. 8 (a)

FIG. 8 (b)

EP 1 821 076 A1

# FIG. 9

EP 1 821 076 A1

# FIG. 10

# FIG. 11

EP 1 821 076 A1

## INTERNATIONAL SEARCH REPORT

| | International application No. |
|---|---|
| | PCT/JP2005/021865 |

**A. CLASSIFICATION OF SUBJECT MATTER**
*G01F1/28*(2006.01), *G01F1/00*(2006.01)

According to International Patent Classification (IPC) or to both national classification and IPC

**B. FIELDS SEARCHED**

Minimum documentation searched (classification system followed by classification symbols)
G01F1/00-9/02

Documentation searched other than minimum documentation to the extent that such documents are included in the fields searched

| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 2003-530150 A (PDS Healthcare Products,<br>Inc.),<br>14 October, 2003 (14.10.03),<br>Par. Nos. [0007], [0060]; Figs. 14, 23, 24<br>& US 6447459 B1      & EP 1267719 A<br>& AU 5321301 A      & CA 2405702 A<br>& CN 1486160 A | 1,3-8,14<br>9-13,15<br>2 |
| Y<br>A | JP 2002-513296 A (Desert Moon Development<br>Limited Partnership),<br>08 May, 2002 (08.05.02),<br>Pages 18 to 28<br>& WO 99/13770 A1 | 9,11,12<br>2 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>05 January, 2006 (05.01.06) | Date of mailing of the international search report<br>17 January, 2006 (17.01.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2005/021865

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2000-28405 A  (Kabushiki Kaisha Nippon Arefu),<br>28 January, 2000 (28.01.00),<br>Claim 1; Fig. 1<br>(Family: none) | 10 |
| Y | JP 1-307670 A  (Mitsubishi Agricultural Machinery Co., Ltd.),<br>12 December, 1989 (12.12.89),<br>Full text; Figs. 11A, 11B<br>(Family: none) | 13 |
| Y | Microfilm of the specification and drawings annexed to the request of Japanese Utility Model Application No. 128416/1984 (Laid-open No. 42902/1986)<br>(Chesuto Kabushiki Kaisha),<br>20 March, 1986 (20.03.86),<br>Full text; Fig. 1<br>(Family: none) | 15 |

Form PCT/ISA/210 (continuation of second sheet) (April 2005)

**EP 1 821 076 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H783713 A
- JP 2000298043 A